# EUROPEAN PATENT SPECIFICATION

(11) **EP 1 858 909 B1**
(45) Date of publication and mention of the grant of the patent: **10.09.2014**
(21) Application number: 06708653.8
(22) Date of filing: 06.03.2006
(51) Int. Cl.: C07H 21/00

(54) **SYNTHESIS OF OLIGONUCLEOTIDES**
OLIGONUKLEOTIDSYNTHESE
SYNTHÈSE D'OLIGONUCLÉOTIDES

(30) Priority: 04.03.2005 EP 05101674; 11.03.2005 US 660549 P
(43) Date of publication of application: 28.11.2007
(73) Proprietor: Girindus AG, 51427 Bergisch-Gladbach (DE)
(72) Inventor: LANGE, Meinolf, 72181 Starzach-felldorf (DE); SCHÖNBERGER, Andreas, 38539 Müden (DE); HOHLFELD, Andreas, 33790 Halle/Westfalen (DE); GRÖSSEL, Olaf, 33790 Halle/Westfalen (DE); KIRCHHOFF, Christina, 33790 Halle/Westfalen (DE); LINK, Fritz, 51429 Bensberg (DE)
(74) Representative: Mross, Stefan P.M.
(86) International application number: PCT/EP2006/060489
(87) International publication number: WO 2006/094963

(56) References cited:
- US-A- 5 532 130
- US-B1- 6 274 725
- NURMINEN, ERKKI J. ET AL: "Nucleophilic and acid catalysis in phosphoramidite alcoholysis" JOURNAL OF THE CHEMICAL SOCIETY, PERKIN TRANSACTIONS 2 , (11), 2159-2165 CODEN: JCSPGI; ISSN: 1472-779X, 2001, XP009050566
- HAYAKAWA, YOSHIHIRO ET AL: "Acid/Azole Complexes as Highly Effective Promoters in the Synthesis of DNA and RNA Oligomers via the Phosphoramidite Method" JOURNAL OF THE AMERICAN CHEMICAL SOCIETY , 123(34), 8165-8176 CODEN: JACSAT; ISSN: 0002-7863, 2001, XP001188947

## Description

### Field of the invention

The present invention relates to methods for preparing oligonucleotides.

### Background of the invention

Oligonucleotides are key compounds in life science having important roles in various fields. They are for example used as probes in the field of gene expression analysis, as primers in PCR or for DNA sequencing.

Furthermore, there are also a number of potential therapeutic applications including i.e. antisense oligonucleotides.

The growing number of applications requires larger quantities of oligonucleotides, therefore, there is an ongoing need for developing improved synthetic method.

For a general overview, see for example "Antisense - From Technology to Therapy" Blackwell Science (Oxford, 1997).

One prominent type of building blocks in the synthesis of oligonucleotides are phosphoramidites; see for example S.L. Beaucage, M. H. Caruthers, Tetrahedron Letters 1859 (1981) 22. These phosphoramidites of nucleosides, deoxyribonucleosides and derivatives of these are commercially available. In normal solid phase synthesis 3'-O-phosphoramidites are used but in other synthetic procedures 5'-O and 2'-O-phosphoramidites are used, too. One step in the preparation of these nucleosides phosphoramidites is the phosphitylating of the (protected) nucleosides. After phosphitylation the prepared amidites are normally isolated by using cost intensive separation methods e.g. chromatography. After isolation the sensitive amidites have to be stocked under special conditions (e.g. low temperature, waterfree). During storage the quality of the amidites may be reduced by a certain degree of decomposition and hydrolysis. Both side reactions can appear and the results are detectable. Most commonly, the hydroxyl group and amino groups and other functional groups present in the nucleoside are protected prior to phosphitylating the remaining 3'-, 5'- or 2'-O hydroxyl group.

These phosphoramidites are then coupled to hydroxyl groups of nucleotides or oligonucleotides. The usage of the isolated amidite can also result in a partial hydrolysis during the amidite coupling.

Phosphoramidites are expensive compounds. Typical prices for deoxyamidites are in the range of € 40,00 per g. The corresponding RNA building blocks are even more expensive.

### Summary of the invention

It is an object of the present invention to provide a method for preparing oligonucleotides overcoming at least some of the drawbacks of prior art.

In one embodiment, the invention provides a method for preparing an oligonucleotide comprising the steps of
a) providing a hydroxyl containing compound having the formula:
   wherein 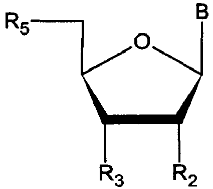
   B is a heterocyclic base
   and
   i) R₂ is H, a protected 2'-hydroxyl group, F, a protected amino group, an O-alkyl group, an O-substituted alkyl, a substituted alkylamino or a C4'-O2'methylen linkage
      R₃ is OR'_{3,} NHR"₃, NR"₃R"'₃, wherein R'₃ is a hydroxyl protecting group, a protected nucleotide or a protected oligonucleotide, R"₃, R"'₃ are independently amine protecting groups,
      and R₅ is OH
      or
   ii) R₂ is H, a protected 2'-hydroxyl group, F, a protected amino group, an O-alkyl group, an O-substituted alkyl, a substituted alkylamino or a C4'-O2'methylen linkage
      R₃ is OH and
      R₅ is OR'₅ and R'₅ is a hydroxyl protecting group, a protected nucleotide or a protected oligonucleotide
      or
   iii)R₂ is OH
      R₃ is OR'_{3,} NHR"₃, NR"₃R"'₃, wherein R'₃ is a hydroxyl protecting group, a protected nucleotide or a protected oligonucleotide, R"₃, R"'₃ are independently amine protecting groups, and
      R₅ is OR'₅ and R'₅ is a hydroxyl protecting group, a protected nucleotide or a protected oligonucleotide 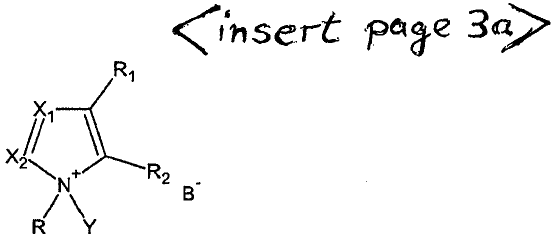 wherein
      R = alkyl, cycloalkyl, aryl, aralkyl, heteroalkyl, heteroaryl
      R₁, R₂ = either H or form a 5 to 6-membered ring together
      X₁, X₂ = independently either N or CH
      Y = H or Si(R₄)₃, with R₄= alkyl, cycloalkyl, aryl, aralkyl, heteroalkyl, heteroa ryl
      B = deprotonated acid
      to prepare a phosphitylated compound
b) reacting said compound with a phosphitylating agent, wherein the phosphitylating agent is represented by the formula: 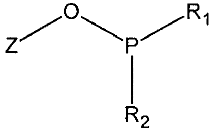 wherein Z represents a leaving group and R₁ and R₂ are independently secondary amino groups,
   in the presence of an activator represented by the formula I (activator I)
c) reacting said phosphitylated compound without isolation with a second compound having the formula 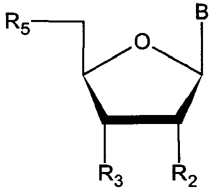 wherein R₅, R₃, R₂, B are independently selected, but have the same definition as above
   in the presence of an activator II selected from the group consisting of tetrazole, derivatives of tetrazole, 4,5-dicyanoimidazole, pyridium-trifluoracetate and mixtures thereof.

According to the invention the phosphitylated compound is prepared by phosphitylating the hydroxyl group of a nucleoside, a nucleotide or an oligonucleotide by using activators having formula I which are preferably derivates of imidazol.

Without purification or isolation, the prepared sensitive phosphoramidite is coupled to hydroxyl groups of nucleosides, nucleotides or oligonucleotides in the presence of an activator II, different from activator I. There is no isolation of the prepared phosphoramidite, no separation of the amidite from activator I. Preferably the reaction is continued in the same reaction vessel. Activator II is used in the presence of activator I.

The prior art activators for amidite coupling have a high reactivity for the activation of the amidite function. Using such an activator for phosphitylation produces also a certain degree of "overreaction" (e.g. 3'-3' by-product). To overcome this and other problems the reactivity of the activator is modulated. In this case the reaction will stop selectively on the amidite level substantially free of by-products, such as 3'-3'-byproduct. Only this result (in-situ generation of the amidite) allows to continue the entire approach by starting with the amidite coupling.

The activator II has the ability to induce the coupling step. After addition of the activator II, the amidite will start with the amidite coupling.

It is possible to use as activator II all activators (different from activator I) which are able to activate the prepared amidite to react with the hydroxyl containing compound of step c); i.e. tetrazole and tetrazole derivatives. Preferred derivatives of tetrazole are benzylmercaptotetrazole and ethylthiotetrazole (ETT). Suitable compounds are selected from the group consisting of Nitrogen-containing heterocycles having in unprotonated form acidic hydrogen, pyridine, pyridine salts and mixtures thereof. The nitrogen-containing hetercycles have an N⁰-H bond, i.e. N is not protonated. These compounds may be used as salts by combing with acids, such as the acids H⁺B⁻ wherein B⁻ has the same meaning as defined in the claims. A further suitable activator II is pyridine, preferably pyridinum trifluoracetate.

Preferred compounds are selected from the group consisting of tetrazole, derivatives of tetrazole, 4,5-Dicyanoimidazole, pyridium trifluoroacetate and mixtures thereof

After coupling, typically oxidation (PO formation) or sulfurisation (PS formation) are used. For the PO formation the peroxide approach is preferred. It is possible to perform this reaction without any extraction steps (iodine oxidation requires a few extraction steps).

In the case of sulfurisation, it is possible to use every known reagent for sulfurisation (i.e. PADS, S-Tetra, beaucage). A preferred reagent for PS formation is sulphur. The difference of production cost is in favour of the use of sulphur.

In one embodiment, the reaction may be in the presence of acetone.

The phosphitylating agent can either be used in a more or less equimolar ratio compared to the hydroxyl groups of the hydroxyl containing compound.

In a further embodiment, it can be used in an excess, e.g. 3 to 5 mol/mol of hydroxyl groups in the hydroxyl containing compound.

In one further preferred embodiment, a polymeric alcohol is added after step b) of claim 1. Suitable polymeric alcohols include polyvinylalcohol (PVA), commercially available as PVA 145000 from Merck, Darmstadt. Preferred are macroporous PVA with a particle size >120 µm (80%). Also membranes with hydroxyl groups or other compounds able to form enols are suitable.

The activator I can be used stoichiometrically, catalytically (3 to 50 mole%, preferably 10 to 30 mole%) or in excess.

In a preferred embodiment, the activator I has a formula selected from the group consisting of 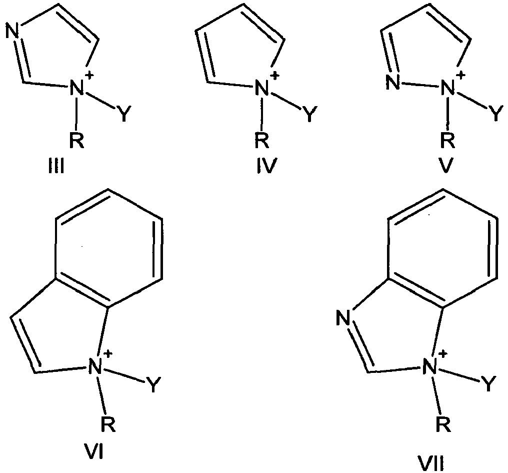 wherein
Y is H or Si(R₄)₃, with R₄= alkyl, cycloalkyl, aryl, aralkyl, heteroalkyl, heteroaryl
B = deprotonated acid
R is methyl, phenyl or benzyl.

The preparation of these activators is for example described in Hayakawa et al, J. Am. Chem. Soc. 123 (2001) 8165-8176.

In one embodiment the activator is used in combination with an additive. Additives can be selected from the unprotonated form of the compounds having formula I and other heterocyclic bases, for example pyridine. Suitable ratios between the activator and the additive are 1:1 to 1:10.

In one preferred embodiment, the activator can be prepared following an "in situ" procedure. In this case the activator will not be isolated, which resulted in improved results of the reaction. Hydrolysis or decomposition of the target molecule is suppressed.

For a high yielding phosphitylation in 3'- and/or 5'-position of oligonucleotides (di, tri, tetra, penta, hexa, hepta and octamers), the in-situ preparation of the activator and the combination with an additive is preferred.

As described above phosphitylating is especially useful in the synthesis of oligonucleotides and the building block phosphoramidites. Therefore, in a preferred embodiment, the hydroxyl containing compound comprises a sugar moiety for example a nucleoside or an oligomer derived therefrom. Such nucleosides are for example adenosine, cytosine, guanosine and uracil, desoxyadenosine, desoxyguanosine, desoxythymidin, desoxycytosine and derivatives thereof, optionally comprising protective groups.

Normally, they will be suitably protected on their heterocyclic functionality and on their hydroxyl bearing groups except of the one that should be phosphitylated. Typically, dimethoxytrityl, monomethoxytrityl or t-butyldimethyl-silyl (TBDMS) are used as protective groups for the 5'OH-group, allowing phosphitylation of the 3'-OH group. Further possible groups are phosphatesters and H-phosphonates, see for example

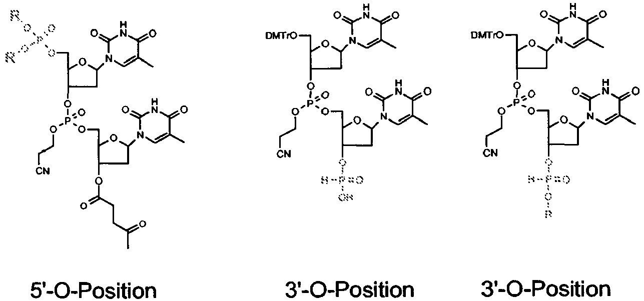

For phosphate ester and phosphodiester, R can be selected from alkyl, aryl, alkylaryl. Phenyl is preferred.

Further hydroxyl protecting groups for 5', 3' and 2' are well-known in the art, e.g. TBDMS.

In general, the phosphitylating agent can be the same as in phosphitylating reactions using 1-H-tetrazole.

In a preferred embodiment, it has the formula 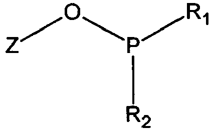 wherein Z represents a leaving group e.g. -CH₂CH₂CN, -CH₂CH=CHCH₂CN, para-CH₂C₆H₄CH₂CN, -(CH₂)₂₋₅N(H)COCF₃, -CH₂CH₂Si(C₆H₅)₂CH₃, or -CH₂CH₂N(CH₃)COCF₃ and R₁ and R₂ are independently secondary amino groups N(R₃)₂, wherein R₃ is alkyl having from 1 to about 6 carbons; or R₃ is a heterocycloalkyl or heterocycloalkenyl ring containing from 4 to 7 atoms, and having up to 3 heteroatoms selected from nitrogen, sulphur, and oxygen.

A typical phosphytilating agent is 2-cyanoethyl-N,N,N',N'-tetraisopropylphosphorodiamidite.

Other preferred phosphitylating reagents are oxazaphospholidine derivatives as described in N. Ok et al., J. Am. Chem. Soc. 2003, 125, 8307 to 8317 incorporated by reference. This phosphitylating agent allows the synthesis of oligonucleotides wherein the internucleotide bond can be converted to phosphorthioates in a stereo selective manner. Such diastereoselective synthesized internucleotidic phosphothioate linkages have promising impact on the use of phosphorthioates as antisense drugs or immunstimulating drugs.

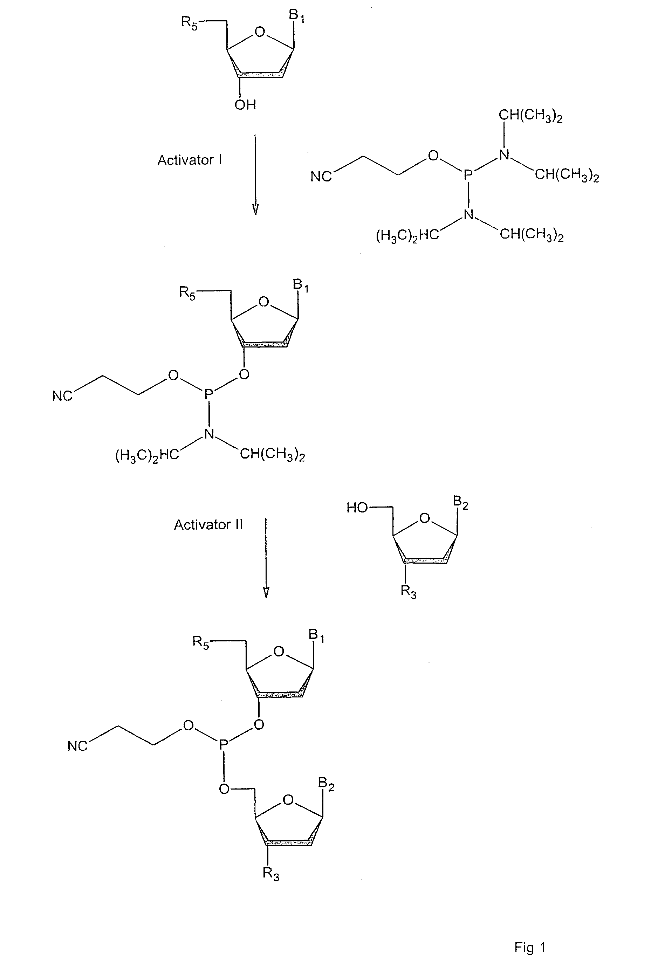

Figure 1 shows a reaction scheme according to the invention.

Suitable examples of depronated acids B⁻ are trifluoroacetat, triflate, dichloroacetat, mesyl, tosyl, o-chlorophenolate. Acids with a pKa below 4.5 are preferred. Preferably, they have a low nucleophilicity.

In one embodiment, the reaction is conducted in the presence of a molecular sieve to dry the reaction medium. In general, water should be excluded or fixed by drying media during reaction.

It is either possible to combine the activator I of the present invention with the phosphitylating agent and add the hydroxyl component later. It is also possible to combine the activator I with the hydroxyl containing compound and add the phosphitylating agent thereafter.

In the case of using an additive, the activator is mixed with the hydroxyl component before the phosphitylating agent is added.

For the "in situ" generation of the activator the selected acid is preferably added after the addition of the additive under controlled reaction temperature.

The phosphitylating agent can be added before the addition of the selected acid or thereafter.

In relation to the addition of acid and phosphitylating agent the nucleoside component can be added at the end or at the beginning.

In a preferred embodiment, the corresponding base of the activator, the hydroxyl containing compound, and the phosphitylating agent are combined and the acid is added to start the reaction.

The phosphitylated compound (phosphoramidite) is then coupled to a hydroxyl group of a nucleoside, a nucleotide or an oligonucleotide in the presence of activator II.

After reacting a compound as described above, the prepared triesters are oxidized. Oxidation may be used to prepare stable phosphate or thiophosphate bonds, for example.

As used herein oligonucleotides covers also oligonucleosides, oligonucleotide analogs, modified oligonucleotides, nucleotide mimetics and the like in the form of RNA and DNA. In general, these compounds comprise a backbone of linked monomeric subunits where each linked monomeric subunit is directly or indirectly attached to a heterocyclic base moiety. The linkages joining the monomeric subunits, the monomeric subunits and the heterocyclic base moieties can be variable in structure giving rise to a plurality of motives for the resulting compounds.

The invention is especially useful in the synthesis of oligonucleotides having the formula Xₙ, wherein each X is selected from A, dA, C, dC, G, dG, U, dT and n = 2 to 30, preferably 2 to 12, more preferably 2 to 8 or 2 to 6 and derivatives thereof comprising protective groups. Modifications known in the art are the modification of the heterocyclic bases, the sugar or the linkages joining the monomeric subunits. Variations of internucleotide linkages are for example described in WO 2004/011474, starting at the bottom of page 11, incorporated by reference.

Typical derivatives are phosphorthioates, phosphorodithioates, methyl and alkyl phosphonates and phosphonoaceto derivatives.

Further typical modifications are at the sugar moiety. Either the ribrose is substituted by a different sugar or one or more of the positions are substituted with other groups such as F, O-alkyl, S-alkyl, N-alkyl. Preferred embodiments are 2'-methyl and 2'-methoxyethoxy. All these modifications are known in the art.

Concerning the heterocyclic base moiety, there are a number of other synthetic bases which are used in the art, for example 5-methyl-cytosine, 5-hydroxy-methyl-cytosine, xanthin, hypoxanthin, 2-aminoadenine, 6- or 2-alkyl derivatives of adenine and guanine, 2-thiouracyl. Such modifications are also disclosed in WO 2004/011474 starting from page 21.

When used in synthesis these bases normally have protecting groups, for example N-6-benzyladenine, N-4-benzylcytosine or N-2-isobutyryl guanine. In general, all reactive groups which are not intended to react in a further reaction have to be protected, especially the hydroxyl groups of the sugar.

In embodiments related to the synthesis of oligonucleotides it is useful to conduct the reaction in the presence of aldehydes or ketones that can be either used as a reaction media or as a co-solvent for other solvents.

Suitable compounds are those that may form enoles. Typical compounds have the formula R₁R₂C = O, wherein R₁ and R₂ are independently H or consist of 1 to 20 carbon atoms which may form cyclic structures alone or R₁ and R₂ form cyclic systems together wherein not both R₁ and R₂ are H. A very preferred ketone is acetone. The presence of acetone quenches the activity of any amount of amines, like diisopropylamine (DIPA), which is liberated during the phosphitylation process. This can be used for the phosphitylation of shorter and longer oligonucleotides with similar results (no decomposition). Other ketone compounds having the formula Rₓ-C(=O)-R_{y} wherein Rₓ and R_{y} are independently C₁-C₆ alkyl or form an cycloalkyl together can also be used as long as they are able to form enolates in the presence of, e.g. amines has a CH₂-group in the α-position.

The invention is further explained by the following non-limiting examples.

### Example 1

### Synthesis of 5'-O-DMTr-T-T-3'-O-Lev cyanoethyl phosphate triester via in-situ preparation of 5'-O-DMTr-T-3'-O-phosphoramidite using Methyl-imidazolium-trifluoroacetate (MIT)

5.0 g 5'-O-DMTr-T-3'-OH (9.2 mmol, 1.0 eq.) and 2.34 g MIT (11.9 mmol, 1.3 eq.) are dissolved in 100 ml dichloromethane and 3 g molecular sieve 3Å is added and the mixture stirred for 10 min. 3.8 ml 2-cyanoethyl N,N,N',N'-tetra-isopropylphosphordiamidite (11.9 mmol, 1.3 eq.) is added. The formation of the 5'-O-DMTr-T-3'-O-phosphoramidite is complete after 2 h. 3.28 g 5'-OH-T-3'-O-Lev (9.64 mmol, 1.05 eq.) and 51 ml tetrazole solution (0.45 M, 22.95 mmol, 2.5 eq) are added and stirred over night. The resulting phosphite triester is oxidized by addition of 4.57 g I₂, 140 ml THF, 35 ml pyridin and 4 ml H₂O. The reaction is complete after 10 min. The reaction mixture is evaporated, dissolved in 300 ml dichloromethane, extracted with 200 ml saturated sodium thiosulfate solution and then extracted with 200 ml saturated sodium hydrogencarbonate solution. The combined aqueous layers are extracted with 30 ml dichloromethane, the combined organic layers are dried over magnesium sulfate and the solvent is evaporated. Yield 9.0 g (colorless foam): 98%; Purity (determined by HPLC): 84%.

### Example 2

### Synthesis of 5'-O-DMTr-dC^{Bz}-T-3'-O-Lev cyanoethyl phosphate triester via in-situ preparation of 5'-O-DMTr-dC^{Bz}-3'-O-phosphoramidite using Methyl-imidazolium-trifluoroacetate (MIT)

108 mg MIT (0.56 mmol, 1.5 eq.) and 224 mg 5'-O-DMTr-dC^{Bz}-3'-OH (0.37 mmol, 1.0 eq.) are dissolved in 9 ml dichloromethane and 300 mg molecular sieve 3Å is added. 140 µl 2-cyanoethyl N,N,N',N'-tetraisopropylphosphordiamidite (0.44 mmol, 1.2 eq.) is added to the stirred solution. The formation of the 5'-O-DMTr-dC^{Bz}-3'-O-phosphoramidite is complete after 30 min. The mixture is filtered and 125 mg 5'-OH-T-3'-O-Lev (0.37 mmol, 1.0 eq.) and 2 ml tetrazole solution (0.45 M, 0.9 mmol, 2.4 eq) are added and stirred over night. The resulting phosphite triester is oxidized by addition of 10 ml oxidizing solution (254 mg I₂, 7.8 ml THF, 1.9 ml pyridin and 222 µl H₂O). The reaction is complete after 30 min. Yield (determined by HPLC): 66%.

### Example 3

### Synthesis of 5'-O-DMTr-dC^{Bz}-dG^{iBu}-3'-O-Lev cyanoethyl phosphorothioate triester via in-situ preparation of 5'-O-DMTr-dc^{Bz}-3'-O-phosphoramidite using Methyl-imidazolium-trifluoroacetate (MIT)

2.57 g 5'-O-DMTr-dC^{Bz}-3'-OH (6.0 mmol, 1.0 eq.) and 1.76 g MIT (9.0 mmol, 1.5 eq.) are dissolved in 6 ml acetone and 6 ml acetonitrile and 3.0 g molecular sieve 3Å is added. 2.46 ml 2-cyanoethyl N,N,N',N'-tetraisopropylphosphor-diamidite (7.74 mmol, 1.3 eq.) is added to the stirred solution. The formation of the 5'-O-DMTr-dC^{Bz}-3'-O-phosphoramidite is complete after 30 min. This solution is filtered and added to a solution of 2.48 g 5'-OH-G^{iBu}-3'-O-Lev (5.7 mmol, 0.95 eq.) and 2.3 g benzylmercaptotetrazole (12.0 mmol, 2.0 eq) in 20 ml dichloromethane and 20 ml acetonitrile and stirred for 30 min. The solution containing the resulting phosphite triester is filtered and sulfurized by addition of 14 g polymer-bound tetrathionate (25.2 mmol, 4.2 eq.). The reaction is complete after 16 h. Yield (determined by HPLC): 84%.

### Example 4

### Synthesis of 5'-O-DMTr-dc^{Bz}-dC^{Bz}-3'-O-Lev cyanoethyl phosphorothioate triester via in-situ preparation of 5'-O-DMTr-dc^{Bz}-3'-O-phosphoramidite using Methyl-imidazolium-trifluoroacetate (MIT)

10 g 5'-O-DMTr-dC^{Bz}-3'-OH (15.8 mmol, 1.0 eq.) and 7.75 g MIT (39.5 mmol, 2.5 eq.) are dissolved in 30 ml dichloromethane and 30 ml acetonitrile, 10 g molecular sieve 3Å is added and the mixture stirred for 30 min. 9.0 ml 2-cyanoethyl N,N,N',N'-tetraisopropylphosphordiamidite (28.4 mmol, 1.8 eq.) are dissolved in 15 ml dichloromethane and 15 ml acetonitrile. The solution of 5'-O-DMTr-dC^{Bz}-3'-OH and MIT is added dropwise to the stirred solution of the 2-Cyanoethyl N,N,N',N'-tetraisopropylphosphordiamidite. The formation of the 5'-O-DMTr-dC^{Bz}-3'-O-phosphoramidite is complete after 30 min. This solution is filtered and added to a solution of 5.43 g 5'-OH-C^{Bz}-3'-O-Lev (12.6 mmol, 0.8 eq.) and 7.6 g benzylmercaptotetrazole (39.5 mmol, 2.5 eq) in 90 ml dimethylformamid and 450 ml acetonitrile and stirred for 10 min. The solution containing the resulting phosphite triester is filtered and sulfurized by addition of 50 g polymer-bound tetrathionate (90 mmol, 5.7 eq.). The reaction is complete after 16 h. Yield (determined by HPLC): 80%.

### Example 5

### Synthesis of 5'-O-DMTr-dA^{Bz}-dG^{iBu}-3'-O-Lev cyanoethyl phosphorothioate triester via in-situ preparation of 5'-O-DMTr-dA^{Bz}-3'-O-phosphoramidite using Methyl-imidazolium-trifluoroacetate (MIT)

5.0 g 5'-O-DMTr-dA^{Bz}-3'-OH (5.8 mmol, 1.0 eq.) and 1.8 g MIT (9.2 mmol, 1.6 eq.) are dissolved in 50 ml acetone and 50 ml acetonitrile, 2.5 g molecular sieve 3Å is added and the mixture stirred for 15 min. 3.0 ml 2-cyanoethyl N,N,N',N'-tetraisopropylphosphordiamidite (9.5 mmol, 1.6 eq.) are added to the stirred solution. The formation of the 5'-O-DMTr-dA^{Bz}-3'-O-phosphoramidite is complete after 1 h. This solution is filtered and added to a solution of 2.22 g 5'-OH-G^{iBu}-3'-O-Lev (5.1 mmol, 0.94 eq.) and 2.9 g benzylmercaptotetrazole (15.1 mmol, 2.6 eq) in 25 ml dichloromethane and 25 ml acetonitrile and stirred for 40 min. The solution containing the resulting phosphite triester is filtered and sulfurized by addition of 2 g polymer-bound tetrathionate (3.6 mmol, 3.9 eq.). The reaction is complete after 16 h. Yield (determined by HPLC): 71%.

### Example 6

### Synthesis of 5'-O-DMTr-T-dG^{iBu}-3'-O-Lev cyanoethyl phosphorothioate triester via in-situ preparation of 5'-O-DMTr-T-3'-O-phosphoramidite using Methyl-imidazolium-trifluoroacetate (MIT)

5.0 g 5'-O-DMTr-T-3'-OH (9.2 mmol, 1.0 eq.) and 2.7 g MIT (13.5 mmol, 1.5 eq.) are dissolved in 50 ml acetone and 50 ml acetonitrile, 2.5 g molecular sieve 3Å is added and the mixture stirred for 15 min. 3.0 ml 2-cyanoethyl N,N,N',N'-tetraisopropylphosphordiamidite (9.5 mmol, 1.03 eq.) are added to the stirred solution. The formation of the 5'-O-DMTr-T-3'-O-phosphoramidite is complete after 1 h. This solution is filtered and added to a solution of 4.44 g 5'-OH-G^{iBu}-3'-O-Lev (10.2 mmol, 1.1 eq.) and 5.3 g benzylmercaptotetrazole (27.6 mmol, 1.6 eq) in 50 ml dichloromethane and 50 ml acetonitrile and stirred for 2 h. The solution containing the resulting phosphite triester is filtered and sulfurized by addition of 30 g polymer-bound tetrathionate (54 mmol, 5.9 eq.). The reaction is complete after 16 h. Yield (determined by HPLC): 90%.

### Example 7

### Synthesis of 5'-O-DMTr-T-dCBz-dCB^{z}-dC^{Bz}-3'-O-Lev cyanoethyl phosphorothioate triester via in-situ preparation of 5'-O-DMTr-T-P(S)-dC^{Bz}-3'-O-phosphoramidite using Methyl-imidazolium-trifluoroacetate (MIT)

100 mg 5'-O-DMTr-T-P(S)-dC^{Bz}-3'-OH (0.10 mmol, 1.0 eq.) and 24.4 mg MIT (0.11 mmol, 1.1 eq.) are dissolved in 10 ml dichloromethane, 200 mg molecular sieve 4Å is added. 32 µl 2-cyanoethyl N,N,N',N'-tetraisopropylphosphor-diamidite (0.10 mmol, 1.0 eq.) is added to the stirred solution. The formation of the 5'-O-DMTr-T-P(S)-dC^{Bz}-3'-O-phosphoramidite is complete after 24 h. 82 mg 5'-OH-dC^{Bz}-3'-P(S)-dC^{Bz}-3'-O-Lev (0.09 mmol, 0.9 eq.) and 366 µl tetrazole solution (0.45 M, 0.16 mmol, 1.6 eq) are added and stirred for 45 h. The resulting phosphite triester is sulfurized by addition of 400 mg polymer-bound tetrathionate within 72 h. Yield (determined by HPLC): 58%.

### Example 8

### Synthesis of 5'-O-DMTr-dC^{Bz}-dG^{iBu}-dC^{Bz}-dC^{Bz}-3'-O-Lev cyanoethyl phosphorothioate triester via in-situ preparation of 5'-O-DMTr-dC^{Bz}-P(S)-dG^{iBu}-3'-O-phosphoramidite using Methyl-imidazolium-trifluoroacetate (MIT)

100 mg 5'-O-DMTr-dC^{Bz}-P(S)-dG^{iBu}-3'-OH (0.09 mmol, 1.0 eq.) and 17.8 mg MIT (0.09 mmol, 1.0 eq.) are dissolved in 10 ml dichloromethane, 200 mg molecular sieve 4Å is added. 28 µl 2-cyanoethyl N,N,N',N'-tetraisopropylphosphordiamidite (0.09 mmol, 1.0 eq.) is added to the stirred solution. The formation of the 5'-O-DMTr-dC^{Bz}-P(S)-dG^{iBu}-3'-O-phosphoramidite is complete after 3 h. 40 mg 5'-OH-dC^{Bz}-3'-P(S)-dC^{Bz}-3'-O-Lev (0.04 mmol, 0.5 eq.) and 0.9 ml ethylthiotetrazole solution (0.25 M, 0.23 mmol, 2.5 eq.) are added and stirred for 2 h. The resulting phosphite triester is sulfurized by addition of 200 mg polymer-bound tetrathionate within 72 h. Yield 30 mg (14.1 µmol, white crystals): 16 %; Purity (determined by HPLC): 67%.

### Example 9

### Synthesis of 5'-O-DMTr-dC^{Bz}-dC^{Bz}-dA^{Bz}-T-3'-O-Lev cyanoethyl phosphorothioate triester via in-situ preparation of 5'-O-DMTr-dC^{Bz}-P(S)-dC^{Bz}-3'-O-phosphoramidite using Benzyl-imidazolium-trifluoroacetate (BIT)

100 mg 5'-O-DMTr-dC^{Bz}-P(S)-dC^{Bz}-3'-OH (0.09 mmol, 1.0 eq.) and 46 mg BIT (0.17 mmol, 1.9 eq.) are dissolved in 5 ml acetone and 5 ml acetonitrile, 500 mg molecular sieve 3Å is added. 58 µl 2-cyanoethyl N,N,N',N'-tetraisopropyl-phosphordiamidite (0.14 mmol, 1.5 eq.) is added to the stirred solution. The formation of the 5'-O-DMTr-dC^{Bz}-P(S)-dC^{Bz}-3'-O-phosphoramidite is complete after 1 h. 41.3 mg 5'-OH-dA^{Bz}-3'-P(S)-T-3'-O-Lev (0.05 mmol, 0.55 eq.) and 43.7 mg benzylmercaptotetrazole (0.23 mmol, 2.5 eq.) are added and stirred for 1.5 h. The resulting phosphite triester is sulfurized by addition of 500 mg polymer-bound tetrathionate within 72 h. Yield (determined by HPLC): 70%.

### Example 10

### Synthesis of 5'-O-DMTr-dG^{iBu}-dG^{iBu}-dG^{iBu}-T-dG^{iBu}-dG^{iBu}-3'-O-Lev cyanoethyl phosphate triester via in-situ preparation of 5'-O-DMTr-dG^{iBu}-P(O)-dG^{iBu}-3'-O-phosphoramidite using Methyl-imidazolium-trifluoroacetate (MIT)

200 mg 5'-O-DMTr-dG^{iBu}-P(O)-dG^{iBu}-3'-OH (0.18 mmol, 1.0 eq.) and 56 mg MIT (0.27 mmol, 1.5 eq.) are dissolved in 5 ml acetone and 300 mg molecular sieve is added. 128 µl 2-cyanoethyl N,N,N',N'-tetraisopropylphosphordiamidite (BisPhos) (0.4 mmol, 2.2 eq.) is added to the stirred solution. The formation of the 5'-O-DMTr-dG^{iBu}-3'-P(O)-dG^{iBu}-3'-O-phosphoramidite is complete after 15 min. 156 mg 5'-OH-dG^{iBu}-T-dG^{iBu}-dG^{iBu}-3'-O-Lev (0.09 mmol, 1.0 eq.) and 87 mg benzylmercaptotetrazole (0.46 mmol, 5.0 eq) are added and stirred for 20 min. The resulting phosphite triester is oxidized by addition of 3.7 ml oxidizing solution (94 mg I₂, 2.9 ml THF, 0.7 ml pyridin and 82 µl H₂O) The reaction is complete after 30 min. Yield (determined by HPLC): 51%.

### Example 11

### Synthesis of 5'-O-DMTr-dG^{iBu}-T-3'-O-Lev cyanoethyl phosphate triester

200 g (312 mmol) DMTr-dG^{iBu}-3'-OH and 80 g (408 mmol) MIT are dissolved in 400 mL dichloromethane and 400 mL acetone. 200 g molecular sieve and 89 mL (1.25 mol) NMI (N-methyl-imidazol) are added. At 15°C 109 mL (344 mmol) BisPhos are added to the stirred solution. The formation of the 5'-O-DMTr-dG^{iBu}-3'-O-phosphoramidite is complete after 10 min. and the solution is allowed to stirr for further 30 min. 88.4 g (260 mmol) 5'-OH-T-3'-O-Lev and 83,4 g (624 mmol) ETT are dissolved with 600 mL acetone and 600 ml dichloromethane. 100 g molecular sieve and 86 mL (1.08 mol) NMI are added. To this stirred solution 800 mL of the phosphoramidite solution are added. The reaction is complete after 10 min and 46 mL butanone peroxide solution (Curox M400) are added to the cooled (ice bath) mixture. The reaction is complete after 5 min. Conversion (determined by HPLC): 100%.

### Example 12

### Synthesis of 5'-O-DMTr-dG^{iBu}-T-3'-O-Lev cyanoethyl phosphorothioate triester

1,0 g (1,56 mmol) DMTr-dG^{iBu}-3'-OH and 368 mg (1,88 mmol) MIT are dissolved in 3 mL dichloromethane and 3 mL acetone. 1 g molecular sieve and 154 µL (1,25 mol) NMI are added. At 15°C 594 µL (1,87 mmol) BisPhos are added to the stirred solution. The formation of the 5'-O-DMTr-dG^{iBu}-3'-O-phosphoramidite is complete after 10 min. and the solution is allowed to stirr for further 30 min. 438 mg (1,29 mmol) 5'-OH-T-3'-O-Lev and 396 mg (3,07 mmol) ETT are dissolved with 5 mL acetone and 5 ml dichloromethane. 1 g molecular sieve and 248 mL (3,61 mol) NMI are added. To this stirred solution 5,5 mL of the phosphoramidite solution are added. The reaction is complete after 10 min and
A) 25 mg (7,8 mmol) sulfur (S₈) and 2,5 mg Na₂Sx9H₂O are added. The reaction is complete after 10 min. Conversion (determined by HPLC): 100%
B) 25 mg (7,8 mmol) sulfur (S₈) are added. The reaction is completed after 3 h. Conversion 99%.

### Example 13

### Synthesis of 5'-O-DMTr-T-dc^{Bz}-dG^{iBu}-T-T-dG^{iBu}-3'-O-Lev cyanoethyl phosphorothioate triester

5,0 g (4,9 mmol) DMTr-T-dC^{Bz}-3'-OH and 2,4 g (12,3 mmol) MIT are dissolved in 10 mL dichloromethane and 10 mL acetone. 8 g molecular sieve and 980 µL (12,3 mol) NMI are added. At 15°C 3,13 mL (9,85 mmol) BisPhos are added to the stirred solution. The formation of the 5'-O-DMTr- T-dC^{Bz} -3'-O-phosphoramidite is complete after 10 min. and the solution is allowed to stirr for further 30 min. 100 mL heptane were added, decanted and 10mL dichloromethane and 10 mL acetone were added to the resulting residue. 4,44 g (2,79 mmol) 5'-OH- dG^{iBu}-T-T-dG^{iBu}-3'-O-Lev and 1,05 g (8,06 mmol) ETT are dissolved with 15 mL acetone and 15 ml dichloromethane. 5 g molecular sieve and 640 µL (8,06 mol) NMI are added. To this stirred solution 20 mL of the phosphoramidite solution are added. The reaction is complete after 10 min and 930 mg (3,09 mmol) PADS are added. The reaction is complete after 10 min. Conversion (determined by HPLC) 92%.

### Example 14

### Synthesis of 5'-O-DMTr-dC^{Bz}-dA^{Bz}- dC^{Bz}-dA^{Bz}-dC^{Bz}-dA^{Bz}- dC^{Bz}-dA^{Bz}-3'-O-Lev cyanoethyl phosphate triester

860 mg (0,45 mmol) 5'-O-DMTr-dC^{Bz}-dA^{Bz}- dC^{Bz}-dA^{Bz}-3'-OH and 133 mg (0,67 mmol) MIT are dissolved in 3 mL dichloromethane and 3 mL acetone. 800 mg molecular sieve and 55 µL (69 mol) NMI are added. 214 µL (0,65 mmol) BisPhos are added to the stirred solution. The formation of the 5'-O-DMTr-dC^{Bz}-dA^{Bz}- dC^{Bz}-dA^{Bz}-3'-3'-O-phosphoramidite is complete after 10 min. and the solution is allowed to stirr for further 20 min. 30 mL heptane were added, decanted and 5 mL dichloromethane and 5 mL acetone were added to the resulting residue. 545 mg (0,3 mmol) 5'-OH-dG^{iBu}-T-T-dG^{iBu}-3'-O-Lev and 117 mg (0,9 mmol) ETT are dissolved with 3 mL acetone, 3 ml dichloromethane and 0,3 mL DMF. 1 g molecular sieve and 70 µL (0,9 mmol) NMI are added. To this stirred solution 8 mL of the phosphoramidite solution are added. The reaction is complete after 30 min and 70 µL butanone peroxide solution (Curox M400) are added to the mixture. The reaction is complete after 10 min. Conversion (determined by HPLC): 80%.

## Claims

1. A method for preparing an oligonucleotide comprising the steps of
a) providing a hydroxyl containing compound represented by the formula : 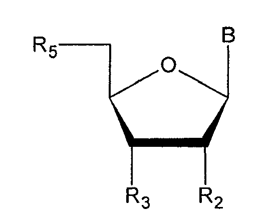 wherein
B is a heterocyclic base
and
i) R₂ is H, a protected 2'-hydroxyl group, F, a protected amino group, an O-alkyl group, an O-substituted alkyl, a substituted alkylamino or a C4'- 02'methylen linkage
R₃ is OR'_{3,} NHR"₃, NR"₃R"'₃, wherein R'₃ is a hydroxyl protecting group, a protected nucleotide or a protected oligonucleotide, R"₃, R"'₃ are independently amine protecting groups,
and R₅ is OH
or
ii) R₂ is H, a protected 2'-hydroxyl group, F, a protected amino group, an O-alkyl group, an O-substituted alkyl, a substituted alkylamino or a C4'- O2'methylen linkage
R₃ is OH and
R₅ is OR'₅ and R'₅ is a hydroxyl protecting group, a protected nucleotide or a protected oligonucleotide
or
iii) R₂ is OH
R₃ is OR'_{3,} NHR"₃, NR"₃R"'₃, wherein R'₃ is a hydroxyl protecting group, a protected nucleotide or a protected oligonucleotide, R"₃, R"'₃ are independently amine protecting groups, and
R₅ is OR'₅ and R'₅ is a hydroxyl protecting group, a protected nucleotide or a protected oligonucleotide
b) reacting said compound with a phosphitylating agent, wherein the phosphitylating agent is represented by the formula :
wherein Z represents a leaving group and R₁ and R₂ are independently 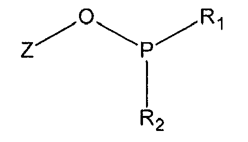
secondary amino groups,
in the presence of an activator represented by the formula I (activator I) 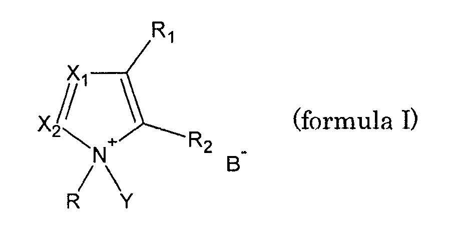
wherein
R = alkyl, cycloalkyl, aryl, aralkyl, heteroalkyl, heteroaryl R₁, R₂ = either H or form a 5 to 6-membered ring together X₁, X₂ = independently either N or CH Y = H or Si(R₄)₃, with R₄= alkyl, cycloalkyl, aryl, aralkyl, heteroalkyl,
heteroaryl
B⁻ = deprotonated acid
to prepare a phosphitylated compound
c) reacting said phosphitylated compound without isolation with a second compound represented by the formula : 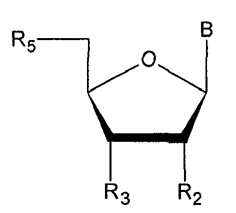 wherein R₅, R₃, R₂, B are independently selected, but have the same definition as above
in the presence of an activator II different from activator I.

2. The method of claim 1, wherein the activator of formula I is represented by a formula selected from the group consisting of 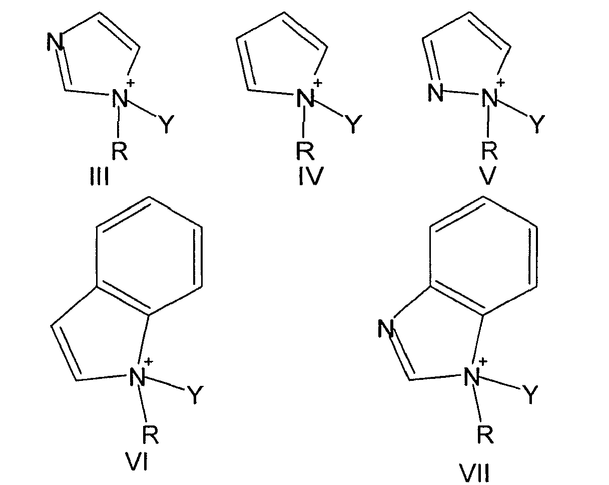 wherein
Y is defined as in claim 1
R is methyl, phenyl or benzyl.

3. The method of any one of claims 1 to 2, wherein the phosphitylating agent is 2-cyanoethyl-N,N,N',N'-tetraisopropylphosphorodiamidite.

4. The method of any one of claims 1 to 3, wherein the deprotonated acid is derived from the group consisting of trifluoroacetic acid, dichloroacetic acid, methane sulfonic acid, trifluormethane sulfonic acid, o-chlorophenolate.

5. The method of any one of claims 1 to 4, wherein the reaction is in the presence of acetone.

6. The method of any one of claims 1 to 5, wherein the phosphitylating agent is used in amount of 1.0 to 1.2 mol/mol of hydroxyl groups in the hydroxyl containing compound.

7. The method of any one of claims 1 to 6, wherein the phosphitylating agent is used in amount of 3 to 5 mol/mol of hydroxyl groups in the hydroxyl containing compound.

8. The method of any one of claims 1 to 7, wherein a polymeric alcohol is added after step b) of claim 1.

9. The method of claim 8, wherein the polymeric alcohol is polyvinyl alcohol.

10. The method of any one of claims I to 9, wherein the deprotonated acid is derived from the group consisting of trifluoroacetic acid, dichloroacetic acid, methane sulfonic acid, trifluormethane sulfonic acid (triflate), o-chlorophenolate and mixtures thereof.

11. The method of any one of claims 1 to 10, wherein the reaction is in the presence of acetone.

12. The method of claim 10 wherein at least 95 % (w/w) of the reaction medium are acetone.

## Patentansprüche

1. Verfahren zur Herstellung eines Oligonukleotids, wobei man in den Verfahrensschritten:
a) eine hydroxylhaltige Verbindung bereitstellt, die durch die Formel: 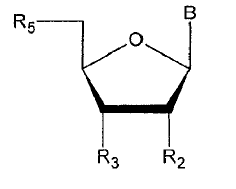 dargestellt wird, wobei
B für eine heterocyclische Base steht
und
i) R₂ für H, eine geschützte 2'-Hydroxylgruppe, F, eine geschützte Aminogruppe, eine O-Alkylgruppe, ein O-substituiertes Alkyl, ein substituiertes Alkylamino oder eine C4'-O2'-Methylen-Verknüpfung steht,
R₃ für OR'₃, NHR"₃, NR"₃R"'₃ steht, wobei R'₃ für eine Hydroxyl-Schutzgruppe, ein geschütztes Nukleotid oder ein geschütztes Oligonukleotid steht, R"₃, R"'3 unabhängig für Amin-Schutzgruppen stehen,
und R₅ für OH steht,
oder
ii) R₂ für H, eine geschützte 2'-Hydroxylgruppe, F, eine geschützte Aminogruppe, eine O-Alkylgruppe, ein O-substituiertes Alkyl, ein substituiertes Alkylamino oder eine C4'-O2'-Methylen-Verknüpfung steht,
R₃ für OH steht und
R₅ für OR'₅ steht und R'₅ für eine Hydroxyl-Schutzgruppe, ein geschütztes Nukleotid oder ein geschütztes Oligonukleotid steht,
oder
iii) R₂ für OH steht,
R₃ für OR'₃, NHR"₃, NR"₃R"'₃ steht, wobei R'₃ für eine Hydroxyl-Schutzgruppe, ein geschütztes Nukleotid oder ein geschütztes Oligonukleotid steht, R"₃, R"'₃ unabhängig für Amin-Schutzgruppen stehen, und
R₅ für OR'₅ steht und R'₅ für eine Hydroxyl-Schutzgruppe, ein geschütztes Nukleotid oder ein geschütztes Oligonukleotid steht,
b) die Verbindung mit einem Phosphitylierungsmittel, das durch die Formel: 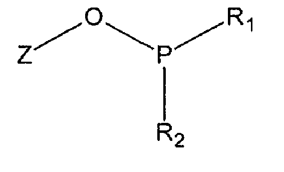 dargestellt wird, wobei Z eine Abgangsgruppe darstellt und R₁ und R₂ unabhängig für sekundäre Aminogruppen stehen,
in Gegenwart eines Aktivators, der durch die Formel I 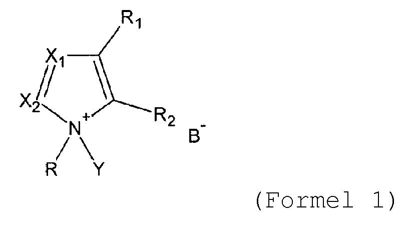 dargestellt wird (Aktivator I), wobei
R = Alkyl, Cycloalkyl, Aryl, Aralkyl, Heteroalkyl, Heteroaryl ist,
R₁, R₂ = entweder H sind oder zusammen einen 5- bis 6-gliedrigen Ring bilden,
X₁, X₂ = unabhängig entweder N oder CH sind Y = H oder Si(R₄)₃ ist, wobei R₄= Alkyl, Cycloalkyl, Aryl, Aralkyl, Heteroalkyl, Heteroaryl ist,
B^{~} = deprotonierte Säure ist,
umsetzt, so dass eine phosphitylierte Verbindung hergestellt wird,
c) die phosphitylierte Verbindung ohne Isolierung mit einer zweiten Verbindung, die durch die Formel: 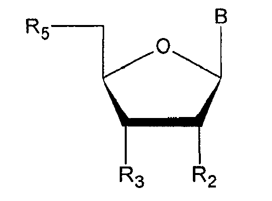 dargestellt wird, wobei R₅, R₃, R₂, B unabhängig ausgewählt sind, jedoch die gleiche Bedeutung wie oben haben,
in Gegenwart eines von Aktivator I verschiedenen Aktivators II umsetzt.

2. Verfahren nach Anspruch 1, wobei der Aktivator der Formel I durch eine aus der aus 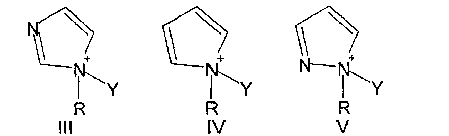 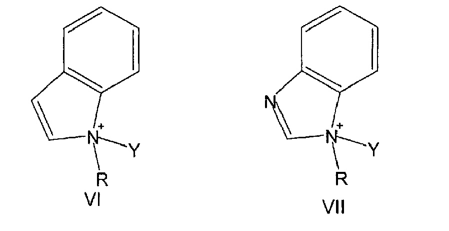 bestehenden Gruppe ausgewählte Formel dargestellt wird, wobei
Y die in Anspruch 1 angegebene Bedeutung hat,
R für Methyl, Phenyl oder Benzyl steht.

3. Verfahren nach einem der Ansprüche 1 bis 2, wobei es sich bei dem Phosphitylierungsmittel um 2-Cyanoethyl-N,N,N',N'-tetraisopropylphosphordiamidit handelt.

4. Verfahren nach einem der Ansprüche 1 bis 3, wobei die deprotonierte Säure aus der aus Trifluoressigsäure, Dichloressigsäure, Methansulfonsäure, Trifluormethansulfonsäure, o-Chlorphenolat bestehenden Gruppe erhalten wird.

5. Verfahren nach einem der Ansprüche 1 bis 4, wobei die Umsetzung in Gegenwart von Aceton erfolgt.

6. Verfahren nach einem der Ansprüche 1 bis 5, wobei das Phosphitylierungsmittel in einer Menge von 1,0 bis 1,2 mol/mol Hydroxylgruppen in der hydroxylhaltigen Verbindung eingesetzt wird.

7. Verfahren nach einem der Ansprüche 1 bis 6, wobei das Phosphitylierungsmittel in einer Menge von 3 bis 5 mol/mol Hydroxylgruppen in der hydroxylhaltigen Verbindung eingesetzt wird.

8. Verfahren nach einem der Ansprüche 1 bis 7, wobei nach Schritt b) in Anspruch 1 ein polymerer Alkohol zugegeben wird.

9. Verfahren nach Anspruch 8, wobei es sich bei dem polymeren Alkohol um Polyvinylalkohol handelt.

10. Verfahren nach einem der Ansprüche 1 bis 9, wobei die deprotonierte Säure aus der aus Trifluoressigsäure, Dichloressigsäure, Methansulfonsäure, Trifluormethansulfonsäure (Triflat), o-Chlorphenolat und Gemischen davon bestehenden Gruppe erhalten wird.

11. Verfahren nach einem der Ansprüche 1 bis 10, wobei die Umsetzung in Gegenwart von Aceton erfolgt.

12. Verfahren nach Anspruch 10, wobei es sich bei wenigstens 95 Gew.-% des Reaktionsmediums um Aceton handelt.

## Revendications

1. Procédé pour préparer un oligonucléotide comprenant les étapes de
a) fourniture d'un composé contenant hydroxyle représenté par la formule : 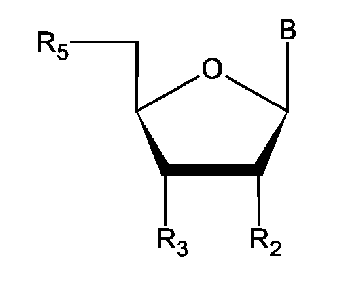 dans laquelle
B est une base hétérocyclique
et
i) R₂ est H, un groupe 2'-hydroxyle protégé, F, un groupe amino protégé, un groupe O-alkyle, un alkyle O-substitué, un alkylamino substitué ou un lieur C4'-O2'méthylène
R₃ est OR'₃, NHR"₃, NR"₃R"'₃, où R'₃ est un groupe protecteur d'hydroxyle, un nucléotide protégé ou un oligonucléotide protégé, R"₃, R"'₃ sont indépendamment des groupes protecteurs d'amine,
et R₅ est OH
ou
ii) R₂ est H, un groupe 2'-hydroxyle protégé, F, un groupe amino protégé, un groupe O-alkyle, un alkyle O-substitué, un alkylamino substitué ou un lieur C4'-O2'méthylène
R₃ est OH et
R₅ est OR'₅ et R'₅ est un groupe protecteur d'hydroxyle, un nucléotide protégé ou un oligonucléotide protégé
ou
iii) R₂ est OH
R₃ est OR'₃, NHR"₃, NR"₃R"'₃, où R'₃ est un groupe protecteur d'hydroxyle, un nucléotide protégé ou un oligonucléotide protégé, R"₃, R"'₃ sont indépendamment des groupes protecteurs d'amine, et
R₅ est OR'₅ et R'₅ est un groupe protecteur d'hydroxyle, un nucléotide protégé ou un oligonucléotide protégé
b) réaction dudit composé avec un agent de phosphitylation, l'agent de phosphitylation étant représenté par la formule : 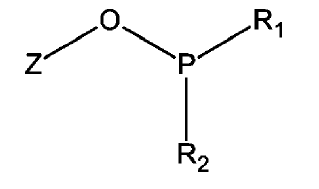 dans laquelle Z représente un groupe partant et R₁ et R₂ sont indépendamment des groupes amino secondaires,
en présence d'un activateur représenté par la formule I (activateur I) 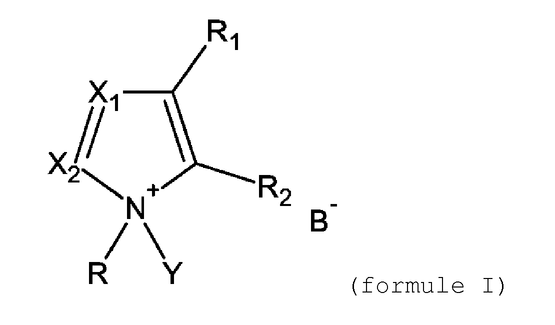 dans laquelle
R = alkyle, cycloalkyle, aryle, aralkyle, hétéroalkyle, hétéroaryle
R₁, R₂ = H ou forment conjointement un cycle de 5 à 6 chaînons
X₁, X₂ = indépendamment N ou CH
Y = H ou Si(R₄)₃, avec R₄ = alkyle, cycloalkyle, aryle, aralkyle, hétéroalkyle, hétéroaryle
B⁻ = acide déprotoné
pour préparer un composé phosphitylé
c) réaction dudit composé phosphitylé sans isolement avec un deuxième composé représenté par la formule : 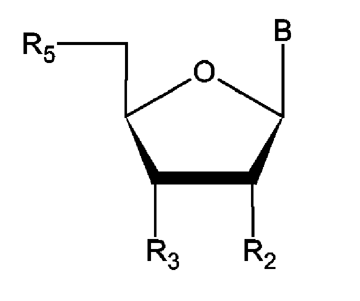 dans laquelle R₅, R₃, R₂, B sont indépendamment choisis, mais ont la même définition que celle décrite ci-dessus en présence d'un activateur II différents de l'activateur I.

2. Procédé de la revendication 1, dans lequel l'activateur de formule I est représenté par une formule choisie dans le groupe constitué de 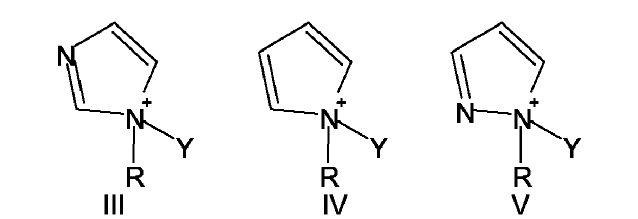 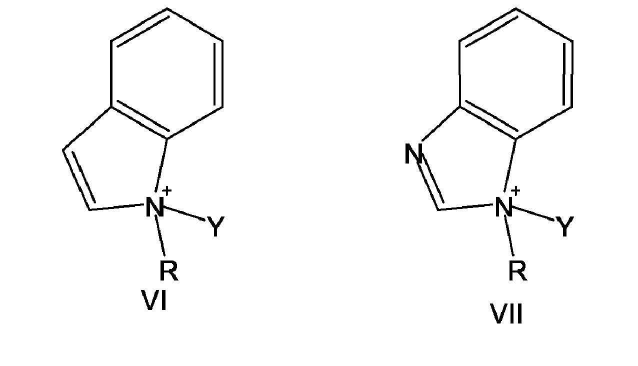 où
Y est tel que défini dans la revendication 1 R est méthyle, phényle ou benzyle.

3. Procédé de l'une quelconque des revendications 1 à 2, dans lequel l'agent de phosphitylation est le 2-cyanoéthyl-N,N,N',N'-tétraisopropylphosphorodiamidite.

4. Procédé de l'une quelconque des revendications 1 à 3, dans lequel l'acide déprotoné est dérivé du groupe constitué de l'acide trifluoroacétique, l'acide dichloroacétique, l'acide méthanesulfonique, l'acide trifluorométhanesulfonique, le o-chlorophénolate.

5. Procédé de l'une quelconque des revendications 1 à 4, dans lequel la réaction est conduite en présence d'acétone.

6. Procédé de l'une quelconque des revendications 1 à 5, dans lequel l'agent de phosphitylation est utilisé en une quantité de 1,0 à 1,2 mol/mol de groupes hydroxyle dans le composé contenant hydroxyle.

7. Procédé de l'une quelconque des revendications 1 à 6, dans lequel l'agent de phosphitylation est utilisé en une quantité de 3 à 5 mol/mol de groupes hydroxyle dans le composé contenant hydroxyle.

8. Procédé de l'une quelconque des revendications 1 à 7, dans lequel un alcool polymère est ajouté après l'étape b) de la revendication 1.

9. Procédé de la revendication 8, dans lequel l'alcool polymère est l'alcool polivinylique.

10. Procédé de l'une quelconque des revendications 1 à 9, dans lequel l'acide déprotoné est dérivé du groupe constitué de l'acide trifluoroacétique, l'acide dichloroacétique, l'acide méthanesulfonique, l'acide trifluorométhanesulfonique (triflate), le o-chlorophénolate et des mélanges de ceux-ci.

11. Procédé de l'une quelconque des revendications 1 à 10, dans lequel la réaction est conduite en présence d'acétone.

12. Procédé de la revendication 10 dans lequel au moins 95 % (m/m) du milieu de réaction sont de l'acétone.
